(19) Europäisches Patentamt / European Patent Office / Office européen des brevets

(11) EP 2 057 995 B1

(12) EUROPÄISCHE PATENTSCHRIFT

(45) Veröffentlichungstag und Bekanntmachung des Hinweises auf die Patenterteilung:
**11.01.2017 Patentblatt 2017/02**

(51) Int Cl.:
***A61K 36/9066*** *(2006.01)* ***A61K 8/97*** *(2017.01)*
***A61Q 19/00*** *(2006.01)* ***A61Q 19/08*** *(2006.01)*

(21) Anmeldenummer: **08162575.8**

(22) Anmeldetag: **19.08.2008**

# (54) Bioaktive Zusammensetzung für kosmetische Anwendungen

Bioactive compound for cosmetic purposes

Composition bioactive pour applications cosmétiques

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.10.2007 DE 102007049612**

(43) Veröffentlichungstag der Anmeldung:
**13.05.2009 Patentblatt 2009/20**

(73) Patentinhaber: **Evonik Degussa GmbH**
**45128 Essen (DE)**

(72) Erfinder:
- **Wenk, Hans, Henning, Dr.**
  **45470, Mülheim an der Ruhr (DE)**
- **Farwick, Mike, Dr.**
  **45138, Essen (DE)**
- **Bergfried, Stefan**
  **45134, Essen (DE)**
- **Maczkiewitz, Ursula**
  **45219, Essen (DE)**

(56) Entgegenhaltungen:
**WO-A2-2007/109210**

- **DATABASE WPI Week 200218 Thomson Scientific, London, GB; AN 2002-131271 XP002619038, & CN 1 319 418 A (GUANGZHOU MEICHEN PHARM CO LTD) 31. Oktober 2001 (2001-10-31)**
- **JAYAPRAKASHA G K ET AL: "Chemical composition of turmeric oil--a byproduct from turmeric oleoresin industry and its inhibitory activity against different fungi", ZEITSCHRIFT FUER NATURFORSCHUNG. SECTION C. BIOSCIENCES,, Bd. 56, Nr. 1-2, 1. Januar 2001 (2001-01-01), Seiten 40-44, XP009143757, ISSN: 0341-0382**
- **LI HONG-XIA ET AL: "Study on ingredients of essential oils of Curcuma wenyujin extracted by supercritical-CO2 fluid extraction and steam distillation", ZHONGGUO ZHONGYAO ZAZHI - CHINA JOURNAL OF CHINESE MATERIA MEDICA, ZHOGGUO YAOXUEHUI, BEIJING, CN, Bd. 31, Nr. 17, 1. September 2006 (2006-09-01), Seiten 1445-1446, XP009143760, ISSN: 1001-5302**
- **JAYAPRAKASHA GUDDADARANGAVVANAHALLY K ET AL: "Evaluation of antioxidant activities and antimutagenicity of turmeric oil: A byproduct from curcumin production", ZEITSCHRIFT FUER NATURFORSCHUNG. C, A JOURNAL OF BIOSCIENCES, TUEBINGEN, DE, Bd. 57, Nr. 9-10, 1. Oktober 2002 (2002-10-01), Seiten 828-835, XP009125905, ISSN: 0939-5075**
- **MARTINS MARIA CELIA ET AL: "Temperature and light effects on turmeric (Curcuma longa L.) oleoresin extracts and curcumin", ARQUIVOS DE BIOLOGIA E TECNOLOGIA, INSTITUTO DE TECNOLOGIA DO PARANA, CURITIBA, BR, Bd. 37, Nr. 4, 1. Januar 1994 (1994-01-01) , Seiten 723-735, XP009143788, ISSN: 0365-0979**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Gebiet der Erfindung

Die Erfindung betrifft die Verwendung eines Extraktes hergestellt nach einem Verfahren zur Herstellung eines Pflanzenextraktes aus *Curcuma* Pflanzen, gekennzeichnet durch die Verfahrensschritte Verfahrensschritt A) Flüssigkeitsextraktion von *Curcuma* Rhizomen, Verfahrensschritt B) optional, Abtrennung eines curcuminoidhaltigen, durch Fällung erhaltenen Feststoffes aus dem in Verfahrensschritt A) erhaltenen Extraktionsgemisches, Verfahrensschritt C) Entfernen vorhandener Lösungsmittel aus dem in Verfahrensschritt A) oder B) erhaltenen Extraktionsgemisch unter Erhalt eines Konzentrates und Verfahrensschritt D) Destillation des Konzentrates bei einem Druck kleiner 1 bar unter Erhalt des Extrakts als Destillat, zur Erhöhung des Feuchtigkeitsgehaltes der Haut, zur Verminderung und Glättung von Hautfalten, zur ebenmäßigen Hautfärbung oder zur Herstellung eines gleichmäßigen Erscheinungsbildes der Hautoberfläche.

Stand der Technik

*Curcuma longa* (Kurkuma, Turmeric) ist eine Pflanze aus der Familie der Ingwergewächse (*Zigimberaceae*), deren Wurzeln und Rhizom aufgrund der intensiven gelben Färbung und des charakteristischen Geschmacks als Farbstoff und Gewürz verwendet wird. Für das getrocknete Rhizom sowie Extrakte hieraus sind verschiedene biologische Wirkungen beschrieben, u. a. Anregung der Magensaftproduktion, krebshemmende, antioxidative und entzündungshemmende Wirkung. *Curcuma longa* wird traditionell in der ayurvedischen Medizin verwendet, unter anderem auch in topischen Anwendungen bei Hauterkrankungen.

Die biologische Wirkung von *Curcuma longa* und Extrakten daraus wird im Allgemeinen auf die Anwesenheit von Curcuminoiden, insbesondere Curcumin, Demethoxycurcumin und Bisdemethoxycurcumin zurückgeführt (vgl. Jayaprakasha et al, J. Agric. Food Chem. 2002, 50(13), 3668-3672; Sharma, Biochemical Pharmacology 1976, 25(15), 1811-1812 und US 5,861,415), denen neben der antioxidativen Wirkung z.B. antiinflammatorische, antibakterielle (Negi et al., J. Agric. Food Chem. 1999,47(10), 4297-4300), pilzhemmende (Apisariyakul et al., Journal of Ethnopharmacology 1995, 49(3), 163-169), antiparasitische, antimutagene, krebshemmende (vgl. z.B. Ruby et al., Cancer Letters 1995, 94(1), 79-83; Soudamini et al., Indian Journal of Pharmacology 1988, 20(2-4), 95-101) und entgiftende Eigenschaften zugeschrieben werden.

Eine charakteristische Eigenschaft von *Curcuma longa* Extrakten ist die intensive Gelbfärbung, die u. a. durch die Curcuminoide hervorgerufen wird. Curcuminoide leiten sich von der Struktur des Curcumins

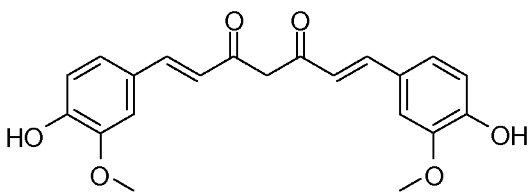

ab, wobei das Substitutionsmuster an den aromatischen Ringen variieren kann. Die bekanntesten Vertreter sind neben Curcumin das Demethoxycurcumin

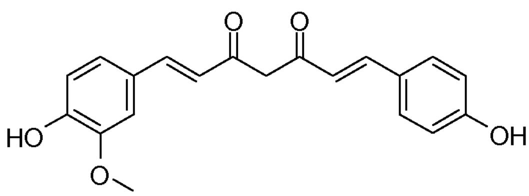

sowie Bisdemethoxycurcumin

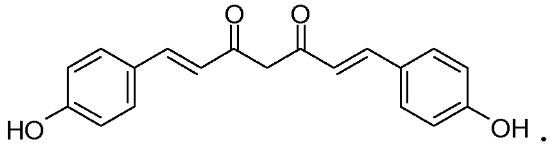

Aufgrund des sehr intensiven Färbevermögens wird Curcumin auch als Lebensmittelfarbstoff (E100) verwendet.

Aufgrund der beschriebenen bioaktiven Wirkungen sind curcuminoidhaltige Extrakte, gewonnen aus z.B. *Curcuma longa,* interessante Kandidaten für Wirkstoffe für topische Anwendungen, insbesondere in kosmetischen Formulierungen. Einer solchen Verwendung steht jedoch die intensive Gelb-/Orangefärbung im Wege, die bereits in Konzentrationen von 0,1 Massen-% eine deutliche, nicht akzeptable Färbung von Cremes oder Lotionen verursacht.

Kim et al. beschreiben die Verringerung der Farbintensität von Curcuma aromatica- und Curcuma longa Extrakten durch Bestrahlung mit Gammastrahlung (Radiation Physics and Chemistry 2006, 75(3), 449-452). Das beschriebene Verfahren ist für die Herstellung kosmetischer Rohstoffe nicht geeignet, da Behandlung mit ionisierender Strahlung im Allgemeinen vom Markt nicht akzeptiert wird.

Aufgabe der Erfindung war es daher, einen Extrakt aus *Curcuma* zur Verfügung zu stellen, der eine deutlich reduzierte Färbung aufweist, jedoch weiterhin die beschriebenen positiven biologischen Wirkungen hat. Die Herstellung sollte darüber hinaus mit kommerziell verfügbaren Verfahren erfolgen, die generell für die Herstellung kosmetischer Rohstoffe akzeptiert sind.

Beschreibung der Erfindung

Es wurde nun überraschenderweise gefunden, dass *Curcuma longa* Extrakte hergestellt durch ein Verfahren wie hierin beschrieben alle gewünschten Eigenschaften aufweisen: So können die entsprechend hergestellten Extrakte problemlos in Konzentrationen bis zu 2 Massen-% und höher in kosmetische Formulierungen eingearbeitet werden, ohne eine erkennbare Färbung hervorzurufen.

Darüber hinaus weist der erfindungsgemäße Extrakt immer noch die erwünschte bioaktive Wirkung auf, insbesondere weist er weiterhin eine starke antioxidative Wirkung auf.

Außerdem wurde gefunden, dass der destillierte Extrakt in der Lage ist, den Feuchtigkeitsgehalt der Haut zu erhöhen, Faltenzahl- und Tiefe der Haut zu verringern und der Haut ein gleichmäßigeres, strahlenderes Erscheinungsbild zu verleihen.

Aufgrund der in der Literatur beschriebenen Zuschreibung der biologischen Wirkungen zu den (stark farbigen) Curcuminoiden waren diese Eigenschaften nicht vorhersehbar.

Das erfindungsgemäße Extrakt und ein Verfahren zu seiner Herstellung werden nachfolgend beschrieben. Bei Angaben in Prozent handelt es sich um Massen-%, wenn nicht anders angegeben.

Gegenstand der Erfindung ist die Verwendung eines Extraktes hergestellt nach einem Verfahren zur Herstellung eines Pflanzenextraktes aus *Curcuma* Pflanzen, gekennzeichnet durch die Verfahrensschritte Verfahrensschritt A) Flüssigkeitsextraktion von *Curcuma* Rhizomen, Verfahrensschritt B) optional, Abtrennung eines curcuminoidhaltigen, durch Fällung erhaltenen Feststoffes aus dem in Verfahrensschritt A) erhaltenen Extraktionsgemisches, Verfahrensschritt C) Entfernen vorhandener Lösungsmittel aus dem in Verfahrensschritt A) oder B) erhaltenen Extraktionsgemisches unter Erhalt eines Konzentrates und Verfahrensschritt D) Destillation des Konzentrates bei einem Druck kleiner 1 bar unter Erhalt des Extrakts als Destillat zur Erhöhung des Feuchtigkeitsgehaltes der Haut, zur Verminderung und Glättung von Hautfalten, zur ebenmäßigen Hautfärbung oder zur Herstellung eines gleichmäßigen Erscheinungsbildes der Hautoberfläche.

Als pflanzlicher Rohstoff für die Herstellung des Extraktes kann das Rhizom von *Curcuma* Pflanzen, bevorzugt von *Curcuma longa* eingesetzt werden, das vor Verfahrensschritt A) vorbehandelt werden kann, so z.B. durch Waschen, Trocknen, Zerkleinern oder Vermahlen. Bevorzugt liegt das Rhizom nach dieser Vorbehandlung als trockenes Pulver vor.

Für Verfahrensschritt A) können die allgemein bekannten Verfahren für die fest-flüssig-Extraktion eingesetzt werden, wie sie z.B. in Ullmann's Encyclopedia of Industrial Chemistry, 7th edition, release 2006 im Kapitel "Liquid-Solid Extraction" und insbesondere im Unterkapitel *"Extraction without chemical reaction"* beschrieben werden. Verfahrensschritt A) kann dabei diskontinuierlich oder kontinuierlich, im Gleich-, Gegen- oder Kreuzstrom erfolgen.

Geeignete Extraktionssmittel für Verfahrensschritt A) sind unpolare bis mäßig polare Lösungsmittel wie z. B. lineare oder verzweigte cyclische oder acyclische Alkane oder Alkene (z.B. Propan, Butan, Pentan, Hexan, Heptan, Cyclohexan, Petrolether), die ggf. mit Halogenen, insbesondere Chlor substituiert sein können, cyclische oder acyclische lineare oder verzweigte Ether (z.B. Diethylether, tert-Butylmethylether, tert-Butylethylether, Tetrahydrofuran), primäre, sekundäre oder tertiäre Alkohole, insbesondere Alkanole (z.B. Ethanol, Isopropanol, n-Butanol, tert-Butanol, Cyclohexanol); Ester kurzkettiger Carbonsäuren mit kurzkettigen Alkoholen (z.B. Ethylacetat, Butylacetat, Ethylacetat), Ketone (z. B. Aceton, Methylisobutylketon), oder Mischungen aus mindestens zwei dieser Lösungsmittel.

Besonders geeignete Lösungsmittel in Verfahrensschritt A) sind verdichtete Gase wie z.B. Propan oder Kohlendioxid, die entweder im subkritischen, nahekritischen oder überkritischen Phasenbereich eingesetzt werden können. Bevorzugt wird in Verfahrensschritt A) eine superkritische Flüssigkeitsextraktion unter Verwendung von verdichtetem Gas, besonders bevorzugt verdichtetem $CO_2$, durchgeführt. Diese ermöglicht eine besonders schonende Entfernung des Extraktionsmittels. Zur Einstellung der Eigenschaften dieser verdichteten Gase können ggf. co-Solventien wie z.B. Ethanol zugesetzt werden. Vorzugsweise kommen Lösungsmittel zum Einsatz, die keine oder nur geringe toxischen oder anderweitig physiologisch nachteiligen Wirkungen haben.

Der Verfahrensschritt A) wird vorzugsweise bei erhöhter Temperatur, vorzugsweise bei einer Temperatur größer 20 °C und bevorzugt bei einer Temperatur von 30 °C bis 80°C durchgeführt.

Ein optionaler Fällungsschritt, der Verfahrensschritt B), kann dem Verfahrensschritt A) nachgeschaltet durchgeführt werden; durch diesen kann eine curcuminoidreiche Feststofffraktion aus dem in A) erhaltenen Produkt abgetrennt werden. Die Fällung kann dabei durch teilweise Verdampfung des Lösemittels (einengen) oder Temperaturverringerung oder Kombinationen daraus erreicht werden. Die Zugabe eines Antisolvens, in dem die Curcuminoide nur eine geringe Löslichkeit aufweisen, kann ebenfalls zur Fällung eingesetzt werden. Vorzugsweise werden hierfür Substanzen eingesetzt, die eine höhere Polarität aufweisen, als das für den Extraktionsschritt verwendete Lösungsmittel. Der Feststoff kann von der verbleibenden Lösung durch übliche Filtrationsverfahren abgetrennt werden, wie sie z.B. in Ullmann's Encyclopedia of Industrial Chemistry, 7th edition, release 2006 im Kapitel "Filtration" beschrieben werden.

In Verfahrensschritt B) kann der Feststoff als Metallkomplex abgetrennt werden. Dazu kann zu dem Extrakt aus Verfahrensschritt A) eine Lösung eines Metallsalzes, wie z.B. Calcium-, Magnesium-, Zink- oder Chromsalz, unter erhöhten Temperaturen hinzugegeben und gemischt werden. Der pH-Wert kann auf einen Vorteilhaften Wert eingestellt werden, um den Metallkomplex erschöpfend zu fällen. Die Temperatur kann unterstützend herabgesetzt werden.

Um die Entfernung des Lösungsmittels in Verfahrensschritt C zur Isolierung des Rohextraktes zu vereinfachen, werden sowohl in Verfahrensschritt A) als auch in Verfahrensschritt B) Extraktions-/Lösungsmittel mit niedrigem Siedepunkt bevorzugt mit einer Siedetemperatur von weniger als 100 °C bei Standardbedingungen eingesetzt.

Die Entfernung vorhandener Extraktions-/Lösungsmittel in Verfahrensschritt C) erfolgt bevorzugt durch Verdampfen. Dieser Verfahrensschritt C) kann bei Umgebungsdruck, Überdruck oder Unterdruck stattfinden, wobei Druck- und Temperaturbedingungen den Eigenschaften des ausgewählten Lösungsmittels oder Lösungsmittelgemisches anzupassen sind. Werden für die Extraktion in Verfahrensschritt A) und die optionale Fällung in Verfahrensschritt B) Lösungsmittel eingesetzt, die bei Normaldruck Siedetemperaturen von über ca. 60 °C aufweisen, so wird die Verdampfung bevorzugt bei vermindertem Druck durchgeführt, wobei Drücke von ca. 100 bis <1000 mbar besonders bevorzugt sind.

Der Verfahrensschritte C) kann dabei entweder absatzweise oder kontinuierlich erfolgen.

Besonders schonend kann die Entfernung des Lösungsmittels im Falle der Verwendung von verdichteten Gasen als Extraktionsmittel erfolgen, da diese bereits durch Entspannen des Überdruckes in den gasförmigen Zustand übergehen und daher auch ohne anlegen eines Unterdruckes bereits bei geringen Temperaturen, z.B. Umgebungstemperatur, verdampft werden können.

Vorzugsweise erfolgt die Entfernung des Lösungsmittels in Verfahrensschritt C) bis zu einem Restgehalt von <1000 ppm Lösungsmittel, wobei Restgehalte von <100 ppm besonders bevorzugt sind.

Pflanzenextrakte sind meist komplexe Naturstoffgemische, deren biologische Eigenschaften häufig durch Synergismen geprägt sind, d. h. die Wirkung des gesamten Extraktes ist stärker als die Summe der Wirkung der einzelnen Substanzen in entsprechender Konzentration. Wenngleich die Abtrennung farbgebender Komponenten aus dem Extrakt zentraler Bestandteil der vorliegenden Erfindung ist, ist eine Konzentration einzelner Substanzen, wie sie bspw. durch fraktionierte Destillation erreicht würde, nicht notwendigerweise von Vorteil. Daher sind für die Destillation Verfahren bevorzugt, die durch eine geringe Trennstufenzahl charakterisiert sind.

Darüber hinaus sind solche Verfahren besonders schonend und daher besonders geeignet, bei denen die Dauer der thermischen Belastung bei der Destillation möglichst kurz ist.

Solche Destillationsverfahren, die eine geringe Anzahl von Trennstufen sowie kurze Kontaktzeiten mit heißen Flächen aufweisen sind z.B. in Dünnschichtverdampfern, Kurzwegverdampfern, Fallfilmverdampfern oder bei der Molekulardestillation realisiert.

Verfahrensschritt D), die Destillation bei einem Druck kleiner 1 bar, erfolgt daher vorzugsweise als Molekulardestillation, besonders bevorzugt als Fallfilmdestillation, Kurzwegverdampfer oder Dünnschichtdestillation, vorzugsweise bei einem Druck von <10 mbar, um eine starke thermische Belastung des Materials weitgehend zu vermeiden. Bevorzugt wird Verfahrensschritt D) bei einem Druck von $10^{-4}$ bis 10 mbar und besonders bevorzugt von ca. $10^{-3}$ bis $10^{-1}$ mbar durchgeführt. Bevorzugt wird Verfahrensschritt D) bei Temperaturen von < 150 °C durchgeführt, besonders bevorzugt sind dabei Temperaturen von 70 bis 130 °C und insbesondere von ca. 100 °C.

Ganz besonders bevorzugt ist eine Durchführung von Verfahrensschritt D) bei einer Temperatur von 97 °C bis 103 °C und bei einem Druck von $10^{-2}$ mbar.

Bevorzugt wird Verfahrensschritt D) bei Bedingungen durchgeführt, unter denen keine neuen Substanzen entstehen,

nachweisbar durch eine GC-Analyse.

In Verfahrensschritt D) kann das Verhältnis von Destillat (erfindungsgemäßer Extrakt) zu Destillationsrückstand (also die Destillationsausbeute) durch die Auswahl der Destillationsparameter gesteuert werden. Hohe Verhältnisse bedeuten dabei hohe Ausbeuten, die entsprechende ökonomische Vorteile bieten, andererseits steigt mit zunehmendem Destillat:Rückstands-Verhältnis auch der Anteil farbgebender Komponenten im Destillat. Als geeignet hat sich ein Destillat:Rückstands-Verhältnis von 50:50 bis 98:2 Gewichtsteilen herausgestellt, wobei Verhältnisse von 70:30 bis 90:10 bevorzugt und Verhältnisse von 80:20 bis 90:10 besonders bevorzugt sind.

Der Extrakt, hergestellt mit dem oben beschriebenen Verfahren besitzt vorzugsweise eine Farbzahl nach Gardner < 6, bevorzugt < 5, besonders bevorzugt < 4.

An Hand der Beispiele wird aufgezeigt, dass der Extrakt sich bei topischer Anwendung auf der Haut durch mehrere positive Eigenschaften bewährt. So kann er den Feuchtigkeitsgehalt der Haut erhöhen so wie Hautfalten glätten und vermindern. Verschiedene Texturparameter der Haut werden vorteilhaft durch Verwendung des erfindungsgemäßen Extraktes beeinflusst, so dass es zu einer ebenmäßigeren Hautfärbung und einer allgemein verbesserten Ausstrahlung der Haut kommt.

Da der beschriebene Extrakt als Antioxidants eine erhebliche Aktivität aufweist, kann dieser als antioxodativer Wirkstoff verwendet werden. Bevorzugt wird der beschriebene Extrakt als antioxidativer Wirkstoff, insbesondere als antioxidativer Wirkstoff zur Verringerung der durch Umweltgifte verursachten oder UV-induzierten Hautschädigung verwendet.

Der beschriebene Extrakt eignet sich aufgrund seiner hellen Farbe und bioaktiven Eigenschaften ausgezeichnet für die Verwendung zur Herstellung einer kosmetischen, Formulierung. Dabei gibt es keine grundsätzlichen Einschränkungen bezüglich der Art der verwendeten Formulierung.
Der beschriebene Extrakt kann aufgrund seiner Öllöslichkeit in Ölphasen ölbasierter Formulierungen oder Emulsionen eingesetzt werden, in Kombination mit geeigneten Solubilisierungsmitteln ist jedoch auch die Verwendung in der wässrigen Phase wasserbasierter Formulierungen oder Emulsionen möglich. So kann der beschriebene Extrakt z.B. in Lotionen und Cremes (z.B. O/W- oder W/O-Emulsionen), Gelformulierungen, Desodorantien (z.B. Sticks, Emulsionen, Pumpsprays, Aerosolsprays, Roll-on Formulierungen), Ölbädern, Schaumbädern, Duschgelen, Shampoos, Haarkonditionierern, Flüssigseifen, Feuchttüchern, Lippenstiften, Foundations, Mundspülungen oder Zahnpasten eingesetzt werden. Besonders geeignet sind sogenannte Leave-on Anwendungen, bei denen ein Verbleiben der Formulierung auf der Haut vorgesehen ist (z.B. Lotionen, Cremes, Desodorantien). Der beschriebene Extrakt kann dabei entweder als alleiniger Wirkstoff oder in Kombination mit weiteren Wirkstoffen verwendet werden.
Die Konzentration des beschriebenen Extraktes in der kosmetischen, dermatologischen oder pharmazeutischen Formulierung ist keinen grundsätzlichen technischen Grenzen unterworfen, jedoch ist bei Konzentrationen oberhalb etwa 5% (abhängig von der Art der Formulierung) ein charakteristischer Geruch des Extraktes deutlich erkennbar. Daher wird der beschriebene Extrakt in den Formulierungen bevorzugt in Konzentrationen von 0,01 bis 5 %, besonders bevorzugt in Konzentrationen von 0,05 bis 1 % eingesetzt.

Die kosmetischen, dermatologischen oder pharmazeutischen Formulierungen sowie die Pflege- und Reinigungsmittel können z.B. mindestens eine zusätzliche Komponenten enthalten, ausgewählt aus der Gruppe der
Emollients,
Emulgatoren und Tenside,
Verdicker/Viskositätsregler/Stabilisatoren,
UV-Lichtschutzfilter,
Antioxidantien und Vitamine,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Biogene Wirkstoffe,
Pflegeadditive,
Überfettungsmittel
Lösungsmittel.

Als Emollients können alle kosmetischen Öle insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen einsetzbar. Des Weiteren eignen sich langkettige Arylsäureester wie z.B. Ester der Benzoesäure, z.B. Benzoesäureester von linearen oder verzweigten, gesättigten oder ungesättigten Alkoholen mit 1 bis 22 C-Atomen, oder auch Benzoesäureisostearylester oder Benzoesäureoctyldocecylester. Weitere als Emollients und Ölkomponenten geeignete Monoester sind z.B. die Methylester und Isopropylester von Fettsäuren mit 12 bis 22 C-Atomen wie z.B. Methyllaurat, Methylstearat, Methyloleat, Methylerucat, Isopropylpalmitat, Isopropylmyristat, Isopropylstearat, Isopropyloleat. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z.B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z.B. im Jojobaöl oder im Spermöl vorliegen. Geeignete Dicarbonsäureester sind z.B. Di-n-butyl-adipat, Di-n-butylsebacat, Di-(2-ethylhexyl)-adipat, Di-(2-hexyldecyl)-succinat, Diisotridecylacelaat. Geeignete Diolester sind z.B. Ethylenglycoldioleat, Ethylenglycol-di-isotridecanoat, Propylenglycol-di-(2-ethylhexanoat), Butandiol-diisostearat, Butandiol-di-caprylat/caprat und Neopentylglycol-di-caprylat. Weitere Fettsäureester, die als Emollients eingesetzt werden können, sind z.B. $C_{12}$-$_{15}$ Alkylbenzoat, Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Haifischlebertran, Dorschleberöl, Walöl, Rindertalg und Butterfett, Wachse wie Bienenwachs, Karnaubapalmwachs, Spermazet, Lanolin und Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl- oder alkoxy- substituierte Polymethylsiloxane oder Cyclomethylsiloxane. Als weitere Ölkörper kommen beispielsweise Guerbetalkohole auf Basis von Fettalkoholen mit 6 bis 18, vorzugsweise 8 bis 10 Kohlenstoffatomen, Ester von linearen C6-C22-Fettsäuren mit linearen C6- C22-Fettalkoholen, Ester von verzweigten C6-C13-Carbonsäuren mit linearen C6-C22-Fettalkoholen, Ester von linearen C6-C22-Fettsäuren mit verzweigten C8-C18-Alkoholen, insbesondere 2-Ethylhexanol oder Isononanol, Ester von verzweigten C6-C13-Carbonsäuren mit verzweigten Alkoholen, insbesondere 2-Ethylhexanol oder Isononanol, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C6-C22-Fettalkoholcarbonate, Guerbetcarbonate, Ester der Benzoesäure mit linearen und/oder verzweigten C6-C22-Alkoholen (z. B. Finsolv™ TN), Dialkylether, Ringöffnungsprodukte von epoxidierten Fettsäureestern mit Polyolen, Siliconöle und/oder aliphatische bzw. naphthenische Kohlenwasserstoffe in Betracht.

Als Emulgatoren oder Tenside können nichtionische, anionische, kationische oder amphotere Tenside eingesetzt werden.

Als nichtionogene Emulgatoren oder Tenside können Verbindungen aus mindestens einer der folgenden Gruppen eingesetzt werden:

Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,

C12/18-Fettsäuremono- und -diester von Anlagerungsprodukten von 1 bis 100 Mol Ethylenoxid an Glycerin,

Glycerinmono- und -diester und Sorbitanmono- und -diester von gesättigten und ungesättigten Fettsäuren mit 6 bis 22 Kohlenstoffatomen und deren Ethylenoxidanlagerungsprodukte, Alkylmono- und -oligoglycoside mit 8 bis 22 Kohlenstoffatomen im Alkylrest und deren Ethylenoxidanlagerungsprodukte,

Anlagerungsprodukte von 2 bis 200 Mol Ethylenoxid an Ricinusöl und/oder gehärtetes Ricinusöl,

Partialester auf Basis linearer, verzweigter, ungesättigter bzw. gesättigter $C_6$-$C_{22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose),
Mono-, Di- und Trialkylphosphate sowie Mono-, Di- und/oder Tri-PEG-alkylphosphate und deren Salze,
Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15,
Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL® EM 90 (Degussa)),
Mischester aus Pentaerythrit, Fettsäuren, Citronensäure und Fettalkohol gemäß DE 11 65 574 und/oder Mischester von Fettsäuren mit 6 bis 22 Kohlenstoffatomen, Methylglucose und Polyolen, wie z.B. Glycerin oder Polyglycerin,
Zitronensäureester wie z.B. Glyceryl Stearate Citrate, Glyceryl Oleate Citrate und Dilauryl Citrate.

Anionische Emulgatoren oder Tenside können wasserlöslich machende anionische Gruppen wie z.B. eine Carboxylat-, Sulfat-, Sulfonat- oder Phosphat-Gruppe und einen lipophilen Rest enthalten. Hautverträgliche anionische Tenside sind dem Fachmann in großer Zahl bekannt und im Handel erhältlich. Dabei kann es sich um Alkylsulfate oder Alkylphosphate in Form ihrer Alkali, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze handeln.
Auch kationische Emulgatoren und Tenside können zugesetzt werden. Als solche können insbesondere quaternäre Ammoniumverbindungen, insbesondere solche, versehen mit mindestens einer linearen und/oder verzweigten, gesättigten oder ungesättigten Alkylkette mit 8 bis 22 C-Atomen, eingesetzt werden, so etwa Alkyltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid eingesetzt werden.
Weiterhin können Monoalklyamidoquats wie z.B. Palmitamidopropyltrimethylammoniumchlorid oder entsprechende Dialkylamidoquats eingesetzt werden.
Weiterhin können biologisch gut abbaubare quaternäre Esterverbindungen eingesetzt werden, bei denen es sich um quaternierte Fettsäureester auf Basis von Mono-, Di- oder Triethanolamin handeln kann. Weiterhin können Alkylguanidiniumsalze als kationische Emulgatoren beigesetzt sein.
Typische Beispiele für milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere beispielsweise auf Basis von Weizenproteinen.
Weiterhin ist es möglich, amphotere Tenside wie z.B. Betaine, Amphoacetate oder Amphopropionate einzusetzen, so z.B. Substanzen wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Von den ampholytischen Tensiden können solche oberflächenaktiven Verbindungen eingesetzt werden, die außer einer C8/18-Alkyl- oder -Acylgruppe im Molekül mindestens eine freie Aminogruppe und mindestens eine-COOH- oder $-SO_3H$- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-Kokosalkylaminopropionat, das Kokosacylaminoethylaminopropionat und das C12/18-Acylsarcosin.

Geeignete Verdicker sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z. B. Carbopole TM oder Synthalene ™), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Verdicker zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Diese anorganischen Ölphasenverdicker können dabei hydrophob modifiziert sein. Zur Verdickung/Stabilisierung von Wasser-in-Öl-Emulsionen können dabei insbesondere Aerosile, Schichtsilikate und /oder Metallsalze von Fettsäuren, wie z.B. Zinkstearat eingesetzt werden.
Als Viskositätsregler für wässrige Tensidsysteme können z.B. NaCl, niedermolekulare nichtionische Tenside, wie Cocoamide DEA/MEA und Laureth-3, oder polymere, hochmolekulare, assoziative, hochethoxylierte Fettderivate, wie PEG-200 Hydrogenated Glyceryl Palmate enthalten sein.

Als UV-Lichtschutzfilter können beispielsweise organische Substanzen eingesetzt werden, die in der Lage

sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z.B. zu nennen:

3-Benzylidencampher und dessen Derivate, z.B. 3-(4-Methylbenzyliden)campher, 4-Aminobenzoesäurederivate, wie z.B. 4-(Dimethylamino)benzoesäure-2-ethylhexylester,4-(Dimethylamino)benzoesäure-2-ethylhexylester und 4-(Dimethylamino)benzoesäureamylester Ester der Zimtsäure, wie z.B. 4-Methoxyzimtsäure-2-ethylhexylester, 4-Methoxyzimtsäureisopentylester, 2-Cyan-3-phenyl-zimtsäure-2-ethylhexylester (Octocrylene), Ester der Salicylsäure, wie z.B. Salicylsäure-2-ethylhexylester, Salicylsäure-4-isopropylbenzylester, Salicylsäurehomomenthylester, Derivate des Benzophenons, wie z.B. 2-Hydroxy-4-methoxybenzophenon, 2-Hydroxy-4-methoxy-4'-methylbenzophenon, 2,2'-Dihydroxy-4-methoxybenzophenon, Ester der Benzalmalonsäure, wie z.B. 4-Methoxybenzmalonsäuredi-2-ethylhexyester, Triazinderivate, wie z.B. 2,4,6-Trianilino-(p-carbo-2'-ethyl-1'-hexyloxy)-1,3,5-triazin und Octyltriazon, Propan-1,3-dione, wie z.B. 1-(4-tert.Butylphenyl)-3-(4'-methoxyphenyl)propan-1,3-dion.

Als wasserlösliche UVB-Lichtschutzfilter kommen in Frage: 2-Phenylbenzimidazol-5-sulfonsäure und deren Alkali-, Erdalkali-, Ammonium-, Alkylammonium-, Alkanolammonium- und Glucammoniumsalze,
Sulfonsäurederivate von Benzophenon, wie z.B. 2-Hydroxy-4-methoxybenzophenon-5-sulfonsäure und ihre Salze, Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.
Als typische UVA-Lichtschutzfilter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden.
Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist.
Weitere geeignete UV-Lichtschutzfilter sind der Übersicht von P. Finkel in SÖFW-Journal 122, 543 (1996) zu entnehmen. Neben den beiden vorgenannten Gruppen primärer UV-Lichtschutzfilter können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.
Als Antioxidantien und Vitamine können z.B. Superoxid-Dismutase, Tocopherol (Vitamin E), Tocopherolsorbat, Tocopherolacetat, andere Ester von Tocopherol, Dibutylhydroxytoluol und Ascorbinsäure (Vitamin C) und ihre Salze sowie deren Derivate (z.B. Magnesium Ascorbylphosphat, Natrium Ascorbylphosphat, Ascorbylsorbat), Ascorbyl Ester von Fettsäuren, butylierte Hydroxybenzoesäure und ihre Salze, Peroxide wie z.B. Wasserstoffperoxid, Perborate, Thioglycolate, Persulfatsalze, 6-Hydroxy-2,5,7,8-Tetramethylchroman-2-Carboxylsäure (TROLOX.RTM), Gallussäure und ihre Alkylester, Harnsäure und ihre Salze und Alkylester, Sorbinsäure und ihre Salze, Liponsäure, Ferulasäure, Amine (z.B. N,N-Diethylhydroxylamin, Amino-Guanidine), Sulfhydryl-Verbindungen (z.B. Glutathion), Dihydroxy-Fumarsäure und ihre Salze, Glycinpidolat, Argininpilolat, Nordihydroguaiaretissche Säure, Bioflavonoide, Curcumin, Lysin, L-Methionin, Prolin, Superoxid Dismutase, Silymarin, Tee Extract, Grapefruit Schalen/Kern Extrakt, Melanin, Rosmarin Extrakt, Thioctansäure, Resveratrol, Oxyresveratrol, etc. eingesetzt werden.
Als Hydrotrope können zur Verbesserung des Fließverhaltens und der Anwendungseigenschaften beispielsweise Ethanol, Isopropylalkohol oder Polyole eingesetzt werden. Polyole, die hier in Betracht kommen, können 2 bis 15 Kohlenstoffatome und mindestens zwei Hydroxylgruppen besitzen. Typische Beispiele sind:

Glycerin Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton, technische Oligoglyceringemische mit einem Eigenkondensationsgrad von 1,5 bis 10 wie etwa technische Diglyceringemische mit einem Diglyceringehalt von 40 bis 50 Gew.-%,
Methylolverbindungen, wie insbesondere Trimethylolethan, Trimethylolpropan, Trimethylolbutan, Pentaerythrit und Dipentaerythrit, Niedrigalkylgucoside, insbesondere solche mit 1 bis 4 Kohlenstoffatomen im Alkylrest, wie beispielsweise Methyl- und Butylglucosid, Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose, Aminozucker, wie beispielsweise Glucamin.

Als Feststoffe können beispielsweise Eisenoxidpigmente, Titandioxid oder Zinkoxidpartikel und die zusätzlich unter "UV-Schutzmittel" genannten eingesetzt werden. Weiterhin können auch Partikel eingesetzt werden, die zu speziellen sensorischen Effekten führen, wie etwa Nylon-12, Bornitrid, Polymerpartikel wie etwa Polyacrylat- oder Polymethylacrylatpartikel oder Siliconelastomere. Einsetzbare Füllstoffe umfassen Stärke und Stärkederivate, wie Tapiocastärke, Distärkephosphat, Aluminium- bzw. Natrium-Stärke, Octenylsuccinat sowie Pigmente, die weder hauptsächlich UV-Filter- noch färbende Wirkung haben, beispielsweise Aerosile® (CAS-Nr. 7631-86-9).

Als Filmbildner zur z.B. Verbesserung der Wasserfestigkeit können beispielsweise eingesetzt werden: Polyurethane, Dimethicone, Copolyol, Polyacrylate oder PVP/VA Copolymer (PVP = Polyvinylpyrrolidon, VA = Vinylacetat). Als fettlösliche Filmbildner können eingesetzt werden: z.B. Polymere auf Basis von Polyvinylpyrrolidon (PVP), Copolymere des Polyvinylpyrrolidons, PVP/Hexadecen-Copolymer oder das PVP/Eicosen-Copolymer.

Als Perlglanzadditive können z.B. Glycoldistearate oder PEG-3 Distearat eingesetzt werden.

Als Deodorantwirkstoffe kommen z.B. Geruchsüberdecker wie die gängigen Parfümbestandteile, Geruchsabsorber, beispielsweise die in der Patentoffenlegungsschrift DE 40 09 347 beschriebenen Schichtsilikate, von diesen insbesondere Montmorillonit, Kaolinit, Ilit, Beidelit, Nontronit, Saponit, Hectorit, Bentonit, Smectit, ferner beispielsweise Zinksalze der Ricinolsäure in Frage. Keimhemmende Mittel sind ebenfalls geeignet, eingearbeitet zu werden. Keimhemmende Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbonilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO® Cosmo P813, Degussa), sowie die in den Patentoffenlegungsschriften DE 198 55 934, DE 37 40 186, DE 39 38 140, DE 42 04 321, DE 42 29 707, DE 42 29 737, DE 42 38 081, DE 43 09 372, DE 43 24 219 und EP 666 732 beschriebenen wirksamen Agenzien.

Als Antitranspirantwirkstoffe können Adstringentien eingesetzt werden, beispielsweise basische Aluminiumchloride wie Aluminiumchlorhydrat ("ACH") und Aluminium-Zirkonium-Glycine-Salze ("ZAG").

Als Insekten-Repellentien können beispielsweise N,N-Diethyl-m-toluamid, 1,2-Pentandiol oder Insect Repellent 3535 eingesetzt werden.

Als Selbstbräuner können z.B. Dihydroxyaceton und Erythrulose eingesetzt werden.

Als Konservierungsstoffe können beispielsweise Mischungen einzelner oder mehrerer Alkylparabenester mit Phenoxyethanol eingesetzt werden. Bei den Alkylparabenestern kann es sich um Methlyparaben, Ethylparaben, Propylparaben und/oder Butylparaben handeln.

Anstelle von Phenoxyethanol können auch andere Alkohole eingesetzt werden, wie beispielsweise Benzylalkohol oder Ethanol. Darüber hinaus können auch andere übliche Konservierungsmittel wie etwa Sorbin- oder Benzoesäure, Salicylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate, Methylisothiazolin, Chlormethyl-isothiazolin, Ethylhexylglycerin oder Caprylyl Glycol eingesetzt werden. Als Konditioniermittel können z.B. organische quaternäre Verbindungen wie Cetrimoniumchlorid, Dicetyldimoniumchlorid, Behentrimoniumchlorid, Distearyldimoniumchlorid, Behentrimoniummethosulfat, Distearoylethyldimoniumchlorid, Palmitamidopropyltrimoniumchlorid, Guar Hydroxypropyltrimoniumchlorid, Hydroxypropylguar Hydroxypropyltrimoniumchlorid, oder Quaternium-80 oder auch Aminderivate wie z.B. Aminopropyldimethicone oder Stearamidopropyldimethylamine verwendet werden.

Als Parfüme können natürliche oder synthetische Riechstoffe oder Gemische daraus eingesetzt werden. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z.B. die Jonone, $\alpha$-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Es können Mischungen verschiedener Riechstoffe eingesetzt werden, die gemeinsam eine ansprechende Duftnote erzeugen. Auch ätherische Öle geringer Flüchtigkeit, die meist als Aromakomponenten eingesetzt werden, eignen sich als Parfüme, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Es können Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller

Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt werden.

Als Farbstoffe können die für kosmetische Zwecke geeigneten und zugelassenen Substanzen eingesetzt werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Polyphenole, Desoxyribonucleinsäure, Coenzym Q10, Retinol, AHA-Säuren, Aminosäuren, Hyaluronsäure, alpha-Hydroxysäuren, Isoflavone, Polyglutaminsäure, Creatin (und Creatinderivate), Guanidin (und Guanidinderivate), Pseudoceramide, essentielle Öle, Peptide, Proteinhydrolysate, Pflanzenextrakte, Bisabolol, Allantoin, Panthenol, Phytantriol, Lakritz Extrakt, Glycyrrhizidin und Idebenon, Skleroglucan, ß-Glucan, Santalbinsäure und Vitaminkomplexe zu verstehen. Beispiele für Pflanzenextrakte sind Kastanien Extrakt, Kamillen Extrakt, Rosmarin Extrakt, schwarze und rote Johannisbeer Extrakt, Birken Extrakt, Hagebutten Extrakt, Algen Extrakte, Grüner Tee Extrakt, Aloe Extrakt, Ginseng Extrakt, Ginko Extrakt, Grapefruit Extrakt, Calendula Extrakt, Kampher, Thymus Extrakt, Mangosteen Extrakt, Cystus Extrakt, Terminalia Arjuna Extrakt, Hafer Extrakt, Oregano Extrakt, Himbeer Extrakt, Erdbeer Extrakt, etc.
Zu den biogenen Wirkstoffen können auch die sogenannten Barriere Lipids gezählt werden, für welche beispielhaft Ceramide, Phytosphingosin und Derivate, Sphingosin und Derivate, Sphinganin und Derivate, Pseudoceramide, Phospholipide, Lysophospholipide, Cholesterin und Derivate, Cholesteryl Ester, freie Fettsäuren, Lanolin und Derivate, Squalan, Squalen und verwandte Substanzen genannt werden. Zu den biogenen Wirkstoffen werden im Sinne der Erfindung auch anti-Akne wie z.B. Benzoylperoxid, Phytosphingosin und Derivate, Niacinamid Hydroxybenzoat, Nicotinaldehyd, Retinolsäure und Derrivate, Salicylsäure und Derrivate, Citronellsäure etc. und anti-Cellulite wie z.B. Xanthin Verbindungen wie Coffein, Theophyllin, Theobromin und Aminophyllin, Carnitin, Carnosin, Salicyloyl Phytosphingosin, Phytosphingosine, Santalbinsäure etc. gezählt, ebenso wie Antischuppenmittel wie beispielsweise Salicylsäure und Derrivate, Zink Pyrithion, Selensulfid, Schwefel, Ciclopiroxolamin, Bifonazol, Climbazol, Octopirox und Actirox etc, ebenso wie Adstringetien wie z.B. Alkohol, Aluminium Derivate, Gallsäure, Pyridoxinsalicylat, Zinksalze wie z.B. Zinksulfat, -acetat, -chlorid, -lactat, Zirconiumchlorohydrate etc. Ebenso können Bleichmittel wie Kojisäure, Arbutin, Vitamin C und Derivate, Hydroquinon, Turmeric oil, Creatinin, Sphingolipide, Niacinamid, etc. zu den biogenen Wirkstoffen gezählt werden.

Als Pflegeadditive können z.B. ethoxylierte Glycerin-Fettsäureester, wie beispielweise PEG-7 Glycerin Cocoate, oder kationische Polymere, wie beispielsweise Polyquaternium-7 oder Polyglycerinester enthalten sein.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Lösungsmittel können z.B. aliphatische Alkohole wie Ethanol, Propanol oder 1,3-Propandiol, cyclische Carbonate wie Ethylencarbonat, Propylencarbonat, Glycerincarbonat, Ester von Mono- oder Polycarbonsäuren wie Ethylacetat, Ethyllactat, Dimethyladipat und Diethyladipat, Propylenglycol, Dipropylenglycol, Glycerin, Glycerincarbonat oder Wasser eingesetzt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll. Folgende Abbildungen sind Bestandteil der Beispiele:

Abbildung 1: Antioxidative Aktivität
Abbildung 2: Ergebnisse der Hautfeuchtigkeitsmessungen (CU = Corneometereinheiten)
Abbildung 3: Oberflächen- und Volumenparameter
Abbildung 4: Änderung der Texturparameter

<u>Beispiele:</u>

*Beispiel 1*

Im Folgenden wird die Herstellung eines destillierten *Curcuma longa* Extraktes durch Dünnschichtdestillation beschrieben.

1 kg getrocknetes, zerkleinertes Rhizom von *Curcuma longa* wurde kontinuierlich für 4 h bei 300 bar und 40 °C erschöpfend mit $CO_2$ extrahiert. Der Rohextrakt wurde durch Expansion des $CO_2$/Extraktgemisches auf Umgebungsdruck in eine Kühlfalle erhalten. Die Extraktion wurde mehrfach wiederholt, wobei Ausbeuten von etwa 45-50 g Extrakt pro kg trockenen Rhizoms erhalten wurden. Der Extrakt ist ein intensiv orangefarbenes Öl mit einer Farbzahl nach

Gardner von 11,3 und mit charakteristischem Geruch.

200 g des Extraktes wurden über einen Dünnschichtverdampfer mit außen liegender Heizfläche bei einer Temperatur von 100 °C und einem Druck von $10^{-2}$ mbar destilliert. Es wurden 162 g einer gelblichen, klaren Flüssigkeit (Farbzahl Gardner 3,5) als Destillat sowie 33 g eines dunklen, trüben Öls als Rückstand erhalten. Der destillierte Extrakt wurde für die im Folgenden beschriebenen Formulierungs- und Wirksamkeitsversuche (Beispiele 3-6) verwendet.

*Beispiel 2*

Das folgende Beispiel verdeutlicht den Zusammenhang zwischen Destillationsausbeute und Farbe des destillierten Extraktes. Die Durchführung erfolgte analog Beispiel 1, jedoch wurde die Temperatur der Verdampferfläche im Bereich von 90 bis 110 °C variiert. Die Ergebnisse sind in der folgenden Tabelle dargestellt:

| | | | |
|---|---|---|---|
| Verdampfertemperatur/°C : | 90 | 100 | 110 |
| Destillationsausbeute/% : | 69 | 74 | 87 |
| Farbzahl (Gardner) : | 3,2 | 3,4 | 3,7 |

*Beispiel 3*

Im Folgenden wird der Einfluss der Zugabe von *Curcuma longa* Rohextrakt bzw. des erfindungsgemäßen destillierten Extraktes zu kosmetischen Formulierungen auf die Farbe dargestellt.

Es wurden verschiedene kosmetische Formulierungen mit dem Rohextrakt sowie mit dem Destillat hergestellt. Außerdem wurde die Leerformulierung ohne Extrakt hergestellt. Von diesen Formulierungen wurde die Farbe bestimmt. Dazu wurde der CIE-Lab-Farbraum zugrunde gelegt. Durch die Angaben der L*a*b* Werte lässt sich eine Farbe genau bestimmen. Die Achsen im Lab-Raum entsprechen unmittelbar wahrnehmbaren Eigenschaften der Farben. Entlang der "a"-Achse sind die Rot(+a)/Grün(-a)-Werte verteilt, entlang der "b"-Achse finden sich die Gelb-(+b)/Blau(-b)-Werte (vgl. DIN 6174).

F1. O/W-Lotion mit 0.5% *Curcuma longa* Extrakt

| | F1-A | F1-B | F1-C |
|---|---|---|---|
| Decyl Oleate | 5,7% | 5,7% | 5,7% |
| Ethylhexyl Stearate | 6,5% | 6,5% | 6,5% |
| Glyceryl Stearate | 0,5% | 0,5% | 0,5% |
| Stearinsäure | 0,7% | 0,7% | 0,7% |
| Cetearyl Glucoside | 1,0% | 1,0% | 1,0% |
| Creatine | 0,5% | 0,5% | 0,5% |
| Glyzerin | 3,0% | 3,0% | 3,0% |
| Kathon CG | 0,015% | 0,015% | 0,015% |
| Wasser | 80,385% | 79,885% | 79,885% |
| Carbomer | 0,2% | 0,2% | 0,2% |
| Ethylhexyl Stearate | 0,8% | 0,8% | 0,8% |
| NaOH (10%) | 0,7% | 0,7% | 0,7% |
| *Curcuma longa* Rohextrakt | - | 0,5% | - |
| *Curcuma longa* dest. | - | - | 0,5% |

F2. O/W-Creme mit 0,5% *Curcuma longa* Extrakt

| | F2-A | F2-B | F2-C |
|---|---|---|---|
| Glyceryl Stearate | 2,5% | 2,5% | 2,5% |
| Stearinsäure | 1,0% | 1,0% | 1,0% |
| Stearylalkohol | 1,5% | 1,5% | 1,5% |

(fortgesetzt)

| | F2-A | F2-B | F2-C |
|---|---|---|---|
| Decyl Cocoate | 8,0% | 8,0% | 8,0% |
| Ethylhexyl Stearate | 7,0% | 7,0% | 7,0% |
| Caprylic-/Caprictriglyceride | 5,0% | 5,0% | 5,0% |
| Cetearyl Glucoside | 1,0% | 1,0% | 1,0% |
| Glyzerin | 3,0% | 3,0% | 3,0% |
| Kathon CG | 0,015% | 0,015% | 0,015% |
| Wasser | 64,235% | 63,735% | 63,735% |
| Carbomer | 0,2% | 0,2% | 0,2% |
| Ethylhexyl Stearate | 0,8% | 0,8% | 0,8% |
| NaOH (10%) | 0,75% | 0,75% | 0,75% |
| Polyglutaminsäure; Hydrolised Sclerotium Gum; Betain; Harnstoff; Kaliumlaktat | 5,0% | 5,0% | 5,0% |
| *Curcuma longa* Rohextrakt | - | 0,5% | - |
| *Curcuma longa* dest. | - | - | 0,5% |

F3. W/O-Lotion mit 0,5% *Curcuma longa* Extrakt

| | F3-A | F3-B | F3-C |
|---|---|---|---|
| Cetyl PEG/PPG-10/1 Dimethicone | 2,0% | 2,0% | 2,0% |
| Ceresin | 0,5% | 0,5% | 0,5% |
| Hydriertes Rizinusöl | 0,5% | 0,5% | 0,5% |
| Decyl Oleate | 9, 0% | 9,0% | 9,0% |
| Caprylic-/Caprictriglyceride | 10,0% | 10,0% | 10,0% |
| Diethylhexyl Ccarbonate | 5,0% | 5,0% | 5,0% |
| PPG-3 Myristyl Ether; Salicyloyl Phytosphingosine | 3,0% | 3,0% | 3,0% |
| Wasser | 69,485% | 68,985% | 68,985% |
| Natriumchlorid | 0,5% | 0,5% | 0,5% |
| Kathon CG | 0,015% | 0,015% | 0,015% |
| *Curcuma longa* Rohextrakt | - | 0,5% | - |
| *Curcuma longa* dest. | - | - | 0,5% |

Die Formulierungen mit dem nicht destillierten Rohextrakt (F1-B, F2-B und F3-B) wiesen eine deutlich erkennbare Gelbfärbung auf, während die mit dem erfindungsgemäßen, destillierten Extrakt hergestellten Formulierungen (F1-C, F2-C und F3-C) nicht von den Leerformulierungen (F1-A, F2-A und F3-A) zu unterscheiden waren. Der optische Eindruck wurde durch Farbmessungen bestätigt.

Die folgende Tabelle gibt die Werte für a* und b* für die verschiedenen Testformulierungen wieder.

| | | ohne Extrakt | Rohextrakt | Destillat |
|---|---|---|---|---|
| Beispiel 1 | a* | -0,7 | -2,5 | -0,8 |
| | b* | 0,5 | 5, 9 | 0,8 |
| Beispiel 2 | a* | -0,8 | -3,2 | -0,9 |
| | b* | 0,8 | 7,7 | 1,1 |
| Beispiel 3 | a* | -0,3 | -2,7 | -0,5 |
| | b* | 0,7 | 7 | 1 |

Vor allem beim b*-Wert erkennt mann eine deutliche Verschiebung in den Gelbbereich, wenn die Formulierung den Rohextrakt enthält. Für die Formulierung mit dem Destillat erhält man dagegen einen b*-Wert, der nahezu identisch ist mit dem der Leerformulierung.

Auch beim a*-Wert zeigt sich mit dem Rohextrakt eine Verschiebung in den Grünbereich. Auch hier zeigen die Leerformulierung und die Formulierung mit dem Destillat nahzu identische Werte.

*Beispiel 4*

Die antioxidative Wirksamkeit der *Curcuma longa* Extrakte wurde mit Hilfe des sogenannten ß-Karotin-Tests untersucht. In diesem Test wird ermittelt, inwieweit ein Antioxidans die gekoppelte Autooxidation von Linolsäure und ß-Karotin hemmt. Diese Reaktion kann photometrisch an Hand des Karotinabbaus verfolgt werden.

Die zu untersuchende Substanz wird 5%ig in Methanol gelöst. In einem weiteren Schritt werden 3 mg ß-Karotin, 400 mg Linolsäure und 4,0 g Tween 40 unter leichtem Erwärmen gemischt, bis das ß-Karotin gelöst ist. Von dieser Mischung solubilisiert man 0,2200g in 25 ml warmem Wasser (50°C). 13 μl der methanolischen Lösung und 1000 μl Solubilisat werden gemischt. Als Kontrolle werden 13 μl Methanol mit 1000 μl Solubilisat gemischt. Anschließend wird die Extinktion dieser Lösungen im einminütigen Abstand bei 470 nm und 50°C gemessen. Die antioxidative Aktivität (AOA) wird als prozentuale Inhibierung bezogen auf die Kontrolle berechnet und zwar für das Zeitintervall von 20-40 min. Die Berechnung der AOA erfolgt nach folgender Formel:

$$AOA = 100 * (DRc-DRs)/(DRc$$

DRc = Abbaurate der Kontrolle
DRs = Abbaurate der Probe

Abbildung 1 gibt die antioxidative Aktivität des Rohextraktes sowie des destillierten Extraktes von *Curcuma longa* wieder.

Das Ergebnis zeigt, dass die Fähigkeit des Extraktes, den Abbau von ß-Karotin zu hemmen, durch die Destillation nicht nennenswert beeinträchtigt wird und dementsprechend auch der erfindungsgemäße, destillierte Extrakt über ausgezeichnete antioxidative Wirkung verfügt.

*Beispiel 5*

Das folgende Beispiel verdeutlicht die Moisturizereigenschaften des destillierten Extraktes, d. h. die Fähigkeit, den Feuchtigkeitsgehalt der Haut zu erhöhen.
Die Bestimmung der Hautfeuchtigkeit erfolgte mit einem Corneometer. Beim Corneometerprinzip wird durch eine Kapazitätsmessung die Hautfeuchtigkeit der "äußeren Schicht" der Oberhaut (Stratum Corneum) bestimmt. Diesem Prinzip liegt die Tatsache der unterschiedlichen Dielektrizitätskonstanten von Wasser und anderen Stoffen zugrunde. Ein entsprechend geformter Messkondensator reagiert auf die in sein Messvolumen eingebrachten Proben mit unterschiedlichen Kapazitätsänderungen, die vom Gerät vollautomatisch erfasst und ausgewertet werden. Die mit Spezialglas beschichtete aktive Sonde wird auf die zu messende Hautstelle gedrückt und nach 1 Sekunde erscheint auf der Anzeige der Corneometermesswert, also der Grad der Feuchtigkeit auf der Hautoberfläche (www.dermatest.de/de/ueberuns.html).

Um den Langzeiteffekt der feuchtigkeitsspendenden Eigenschaften des Destillates zu untersuchen, wurde eine vierwöchige Studie mit 30 Probanden durchgeführt. Die Bestimmung der Hautfeuchtigkeit erfolgte mit einem Corneometer CM 825 (Courage & Khazaka). Vor jeder Messung mussten sich die Probanden mindestens 15 min in einem klimatisierten Raum aufhalten (21-22°C, 55% r.H.).

Die Probanden wurden in zwei Gruppen aufgeteilt. Die erste Gruppe erhielt die Testformulierung mit 0.5% des erfindungsgemäßen, destillierten Extraktes (F4-B), die zweite Gruppe erhielt die Formulierung ohne Wirkstoff (F4-A). Diese Formulierungen mussten auf der Innenseite des Unterarmes zweimal täglich auftragen werden. Vor Beginn der Anwendung sowie nach 4 Wochen wurde die Hautfeuchtigkeit gemessen. Es wurde die Differenz der Corneometereinheiten zum Startwert berechnet (ΔCU).

Die Testformulierung hatte folgende Zusammensetzung:

Testformulierung F4

| | F4-A | F4-B |
|---|---|---|
| Polyglyceryl-3 Methylglucose Distearate | 3,0% | 3,0% |

(fortgesetzt)

| | F4-A | F4-B |
|---|---|---|
| Glyceryl Stearate | 2,0% | 2,0% |
| Stearylalkohol | 1,0% | 1,0% |
| C12-25 Alkyl Benzoate | 9,5% | 9,5% |
| PPG-3 Myristyl Ether | 9,5% | 9,5% |
| Glycerin | 3,0% | 3,0% |
| Phenoxyethanol, Methylparaben, Ethylparaben, Butylparaben, Propylparaben, Isobutylparaben | 0,5% | 0,5% |
| Parfüm | 0,1% | 0,1% |
| *Curcuma longa* Dest. | 0% | 0,5% |
| Wasser | ad | ad |
| | 100,0% | 100,0% |

Die Ergebnisse der Hautfeuchtigkeitsmessungen sind in Abbildung 2 dargestellt. (CU = Corneometereinheiten) Mit der Leerformulierung (Vehikel, F4-A) zeigt sich eine schwache Verbesserung der Hautfeuchtigkeit nach vierwöchiger Anwendung. Dieser Effekt ist üblich, da bereits z.B. der pflegende Effekt der in der Formulierung enthaltenen Öle zu einer leicht erhöhten Hautfeuchtigkeit führt. Im Gegensatz dazu wurde in der Gruppe, die eine Formulierung mit 0,5% des erfindungsgemäßen Extraktes (F4-B) applizierte, die Hautfeuchtigkeit stark erhöht.

*Beispiel 6*

Einfluss des *Curcuma longa* Destillats auf die Hautoberfläche

Die Charakterisierung der Hautoberfläche erfolgte mit einer speziellen Kamera, der Visisoscan VC 98 von Courage & Khazaka. Diese Kamera besitzt einen hochauflösenden schwarz-weiß Videosensor und eine ringförmige UV-A-Lichtquelle zur gleichmäßigen Ausleuchtung der Hautoberfläche. Mit dieser Kamera wird ein stark vergrößertes Bild der Hautoberfläche aufgenommen. Mit Hilfe einer speziellen Software werden dann verschiedene Parameter, die den Zustand der Hautoberfläche beschreiben, berechnet.

Der Parameter Oberfläche beschreibt die Größe der welligen Oberfläche des Hautbildes im Verhältnis zur flachen, geplätteten Ebene. Je weniger faltig die Hautoberfläche ist, desto geringer ist also der Messwert.

Für den Parameter Volumen wird das Volumen berechnet, das erforderlich wäre, wollte man die Falten mit Flüssigkeit auffüllen. Je mehr Falten vorhanden sind und je tiefer diese Falten sind, desto größer ist der Parameter Volumen.

Außerdem berechnet die Software noch sogenannte Texturparameter, die sich auch Farbunterschiede zwischen Nachbarpixeln beziehen. Es handelt sich hierbei um die Parameter Energie, Varianz, Kontrast, Entropie und Homogenität. Zusammenfassend beschreiben sie die Gleichmäßigkeit der Hautoberfläche. Eine Verbesserung dieser Parameter bedeutet ein ebenmäßigeres, strahlenderes Hautbild.

Es wurde eine achtwöchige Studie mit 30 Probanden durchgeführt. Die Probanden wurden in 2 Gruppen eingeteilt. Die erste Gruppe erhielt die Testformulierung mit 0.5% der erfindungsgemäßen Verbindung(F4-B), die zweite Gruppe erhielt die Formulierung ohne Wirkstoff (F4-A). Diese Formulierungen mussten auf der Innenseite eines Unterarmes zweimal täglich auftragen werden. Vor Beginn der Anwendung sowie nach 8 Wochen wurde mit der Kamera ein Bild der Hautoberfläche aufgenommen und ausgewertet. Für die verschiedenen Parameter wurde anschließend die prozentuale Differenz zwischen Startwert und 8-Wochenwert berechnet.

Abbildung 3 gibt die Verbesserung der Parameter Oberfläche und Volumen wieder.

Nach achtwöchiger Anwendung der Leerformulierung (F4-A) wird wiederum die bereits beschriebene nur leichte Verbesserung beobachtet. Die Formulierung F4-B mit 0,5% des destillierten Extraktes führte hingegen zu einer starken, signifikanten Verbesserung. Die erfindungsgemäße Verbindung ist demzufolge in der Lage, Faltenanzahl und Faltentiefe zu verringern.

Abbildung 4 zeigt die prozentuale Änderung der Texturparameter.

Mit der Leerformulierung (F4-A) konnte nur eine leichte Verbesserung des Hautbildes erzielt werden. Nach Applikation der Formulierung F4-B mit 0,5% des erfindungsgemäßen Extraktes wurde eine starke Verbesserung aller beobachteten Parameter erreicht. Der destillierte *Curcuma longa* Extrakt führt also zu einer deutlich ebenmäßigeren, strahlenderen Hautoberfläche.

## Patentansprüche

1. Verwendung eines Extraktes hergestellt nach einem Verfahren zur Herstellung eines Pflanzenextraktes aus *Curcuma* Pflanzen, **gekennzeichnet durch** die Verfahrensschritte:

   Verfahrensschritt A) Flüssigkeitsextraktion von *Curcuma* Rhizomen
   Verfahrensschritt B) optional, Abtrennung eines curcuminoidhaltigen, **durch** Fällung erhaltenen Feststoffes aus dem in Verfahrensschritt A) erhaltenen Extraktionsgemisches
   Verfahrensschritt C) Entfernen vorhandener Lösungsmittel aus dem in Verfahrensschritt A) oder B) erhaltenen Extraktionsgemisch unter Erhalt eines Konzentrates und
   Verfahrensschritt D) Destillation des Konzentrates bei einem Druck kleiner 1 bar unter Erhalt des Extrakts als Destillat,

   zur Erhöhung des Feuchtigkeitsgehaltes der Haut, zur Verminderung und Glättung von Hautfalten, zur ebenmäßigen Hautfärbung oder zur Herstellung eines gleichmäßigen Erscheinungsbildes der Hautoberfläche.

2. Verwendung nach Anspruch 1, **dadurch gekennzeichnet, dass** in Verfahrensschritt A) eine superkritische Flüssigkeitsextraktion unter Verwendung von verdichtetem Gas durchgeführt wird.

3. Verwendung nach mindestens einem der Ansprüche 1 oder 2, **dadurch gekennzeichnet, dass** in Verfahrensschritt D) eine Molekulardestillation eingesetzt wird.

4. Verwendung nach mindestens einem der Ansprüche 1 bis 3, **dadurch gekennzeichnet, dass** in Verfahrensschritt D) eine Fallfilmdestillation, eine Kurzwegverdampfung oder eine Dünnschichtdestillation mit einem Druck <10 mbar ausgeführt wird.

## Claims

1. Use of an extract prepared by a process for the preparation of a plant extract from *Curcuma* plants, **characterized by** the process steps:

   process step A) liquid extraction of *Curcuma* rhizomes
   process step B) optionally, separation of a curcuminoid-containing solid obtained by precipitation from the extraction mixture obtained in process step A)
   process step C) removal of solvents present from the extraction mixture obtained in process step A) or B) to obtain a concentrate and
   process step D) distillation of the concentrate at a pressure of less than 1 bar to give the extract as distillate,

   for increasing the moisture content of the skin, for reducing and smoothing wrinkles in the skin, for even skin coloration or for producing an even appearance of the skin surface.

2. Use according to Claim 1, **characterized in that**, in process step A), a supercritical liquid extraction is carried out using compressed gas.

3. Use according to at least one of Claims 1 or 2, **characterized in that** a molecular distillation is used in process step D).

4. Use according to at least one of Claims 1 to 3, **characterized in that**, in process step D), a falling-film distillation, a short-path evaporation or a thin-film distillation is executed with a pressure < 10 mbar.

## Revendications

1. Utilisation d'un extrait confectionné selon un procédé de fabrication d'un extrait végétal de plantes *curcuma,* **caractérisée par** les étapes de procédé suivantes :

   A) extraction liquide de rhizomes de *curcuma,*
   B) facultativement, séparation d'un solide contenant des curcuminoïdes obtenu par précipitation à partir du

mélange d'extraction obtenu à l'étape de procédé A),
C) élimination des solvants présents dans le mélange d'extraction obtenu aux étapes de procédé A) et B) pour obtenir un concentré, et
D) distillation du concentré sous une pression inférieure à 1 bar pour obtenir un distillat de l'extrait,

pour augmenter l'humidité de la peau, réduire et lisser les rides, égaliser la coloration de la peau ou produire un aspect régulier de la surface de la peau.

2. Utilisation selon la revendication 1, **caractérisée en ce qu'**on réalise une extraction par phase supercritique à l'étape de procédé A) en utilisant un gaz comprimé.

3. Utilisation selon au moins une des revendications 1 ou 2, **caractérisée en ce qu'**on utilise une distillation moléculaire à l'étape de procédé D).

4. Utilisation selon au moins une des revendications 1 à 3, **caractérisée en ce qu'**on réalise une distillation à ruissellement, une évaporation moléculaire ou une distillation sur couche mince sous une pression < 10 mbar.

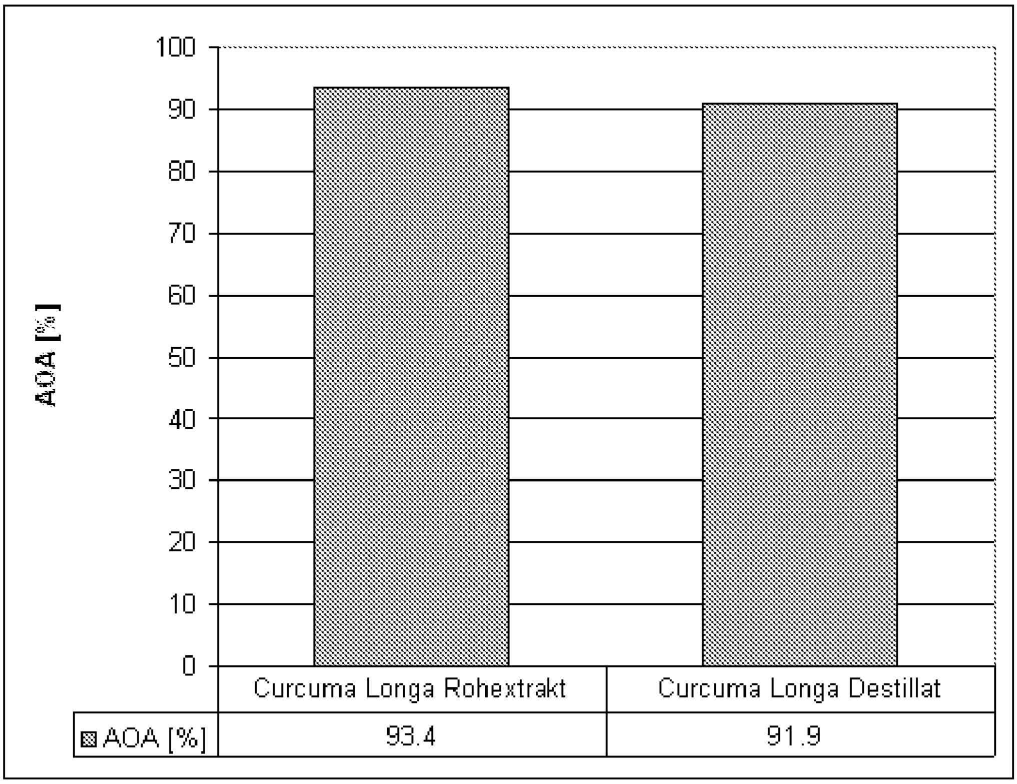
100
90
80
70
60
50
40
30
20
10
0
AOA [%]
Curcuma Longa Rohextrakt
Curcuma Longa Destillat
AOA [%]
93.4
91.9

Abbildung 1

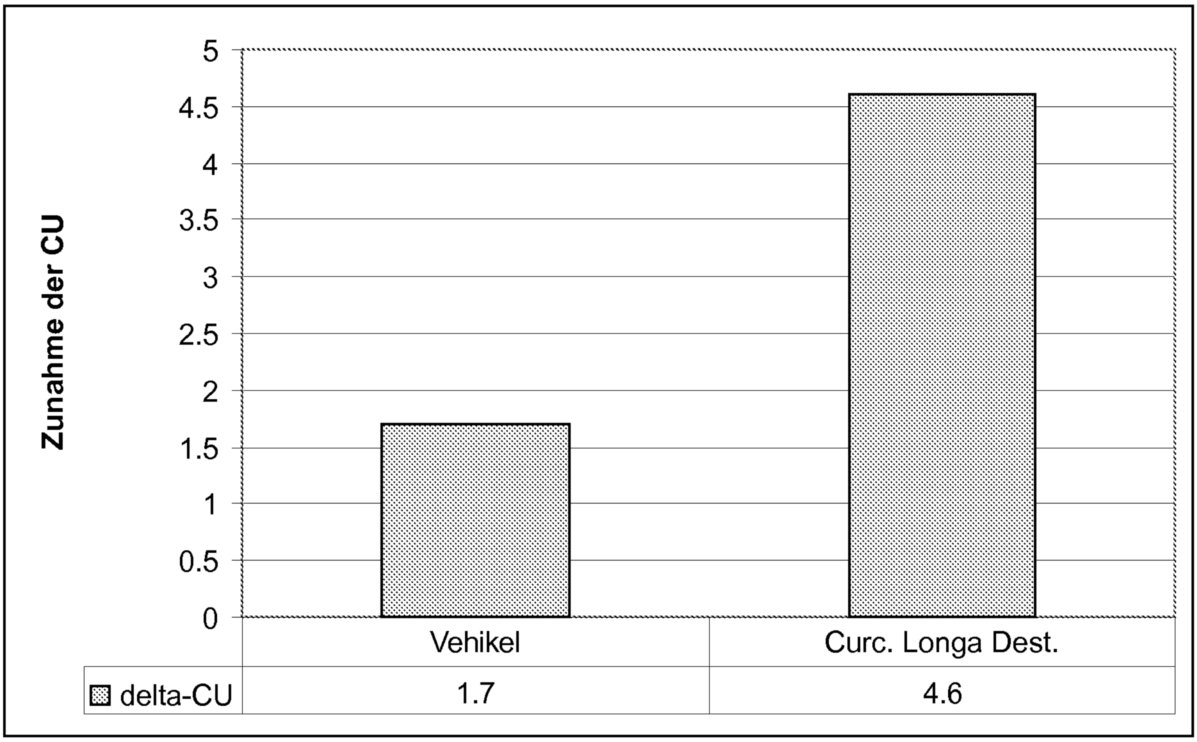
Zunahme der CU
5
4.5
4
3.5
3
2.5
2
1.5
1
0.5
0
Vehikel
Curc. Longa Dest.
delta-CU
1.7
4.6

Abbildung 2

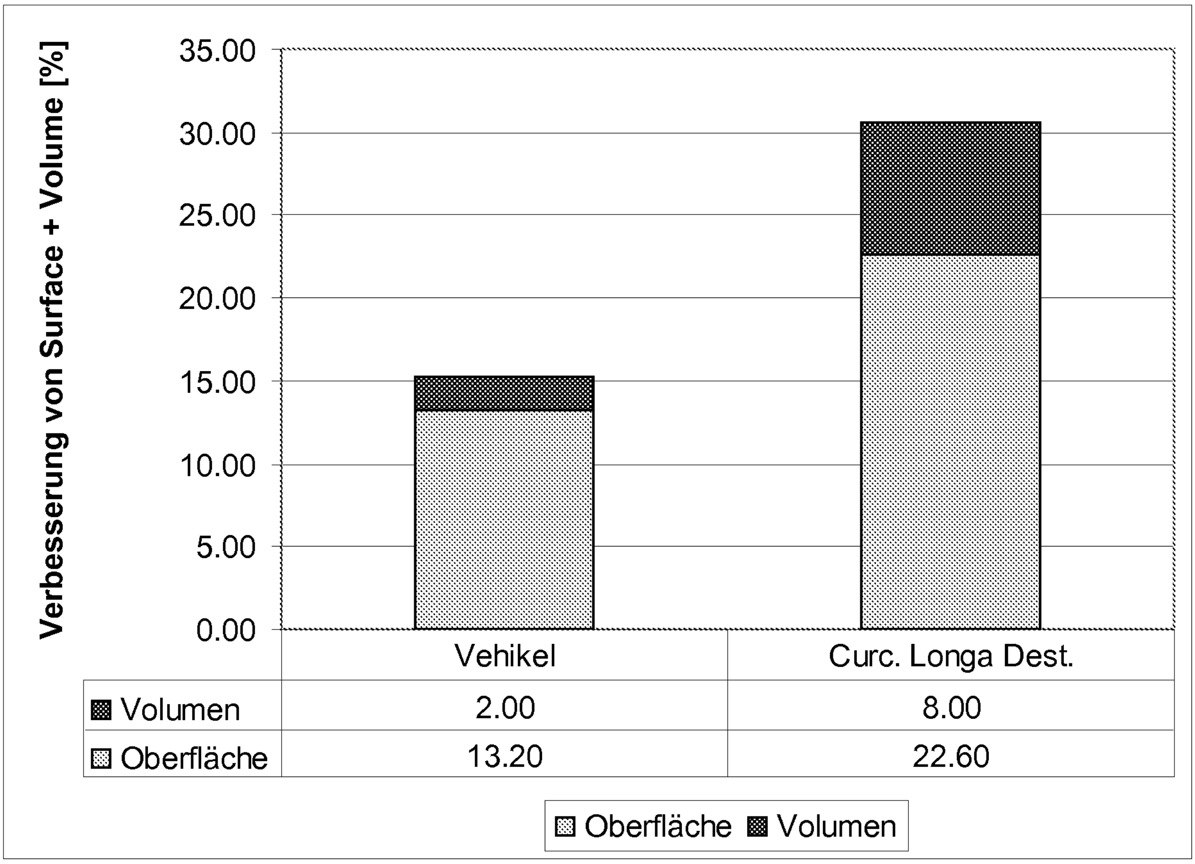
Verbesserung von Surface + Volume [%]
35.00
30.00
25.00
20.00
15.00
10.00
5.00
0.00
Vehikel
Curc. Longa Dest.
Volumen
2.00
8.00
Oberfläche
13.20
22.60
Oberfläche
Volumen

Abbildung 3

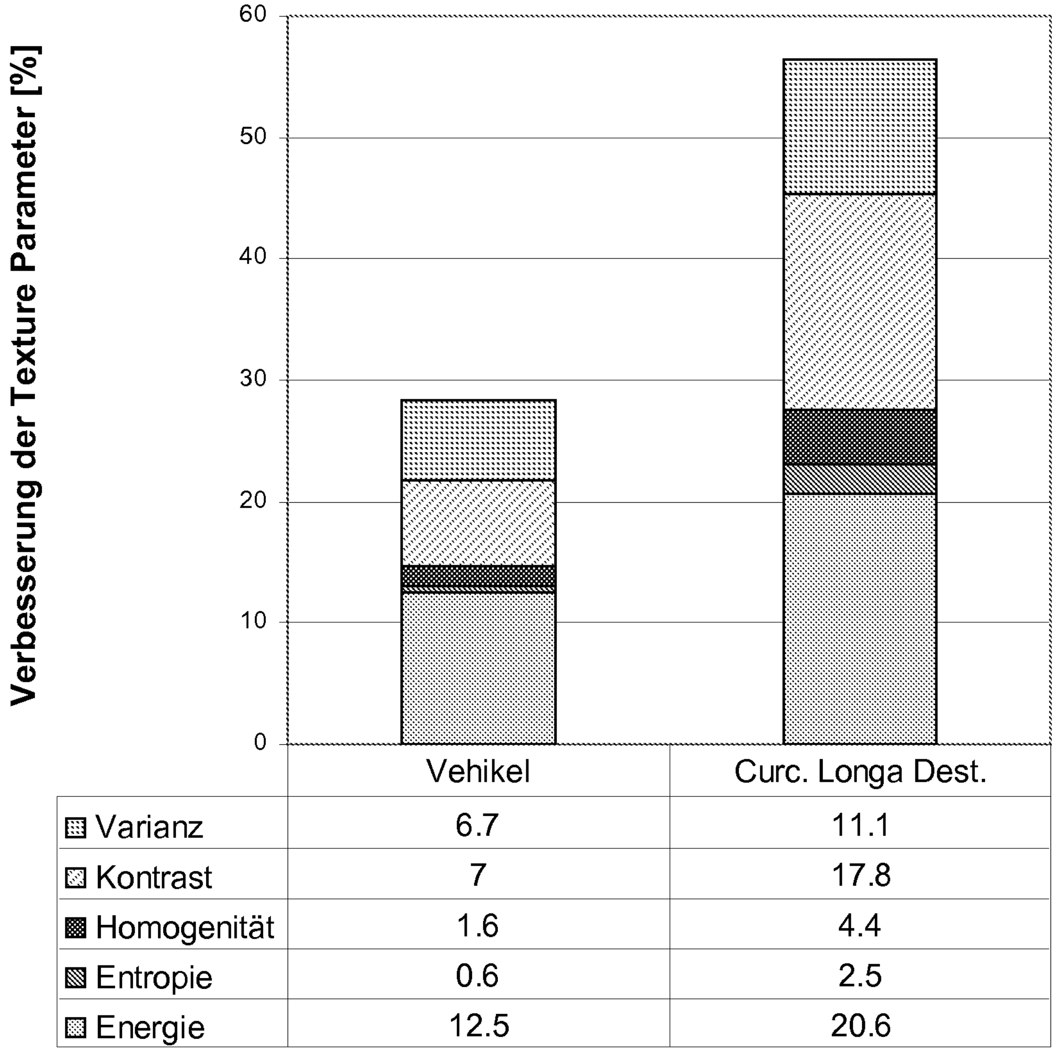


| | Vehikel | Curc. Longa Dest. |
|---|---|---|
| Varianz | 6.7 | 11.1 |
| Kontrast | 7 | 17.8 |
| Homogenität | 1.6 | 4.4 |
| Entropie | 0.6 | 2.5 |
| Energie | 12.5 | 20.6 |

Energie Entropie Homogenität Kontrast Varianz

Abbildung 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5861415 A
- DE 1165574
- DE 4009347
- DE 19855934
- DE 3740186
- DE 3938140
- DE 4204321
- DE 4229707
- DE 4229737
- DE 4238081
- DE 4309372
- DE 4324219
- EP 666732 A


### In der Beschreibung aufgeführte Nicht-Patentliteratur


- **JAYAPRAKASHA et al.** *J. Agric. Food Chem.,* 2002, vol. 50 (13), 3668-3672
- **SHARMA.** *Biochemical Pharmacology,* 1976, vol. 25 (15), 1811-1812
- **NEGI et al.** *J. Agric. Food Chem.,* 1999, vol. 47 (10), 4297-4300
- **APISARIYAKUL et al.** *Journal of Ethnopharmacology,* 1995, vol. 49 (3), 163-169
- **RUBY et al.** *Cancer Letters,* 1995, vol. 94 (1), 79-83
- **SOUDAMINI et al.** *Indian Journal of Pharmacology,* 1988, vol. 20 (2-4), 95-101
- **KIM et al.** beschreiben die Verringerung der Farbintensität von Curcuma aromatica- und Curcuma longa Extrakten durch Bestrahlung mit Gammastrahlung. *Radiation Physics and Chemistry,* 2006, vol. 75 (3), 449-452
- Liquid-Solid Extraction. Ullmann's Encyclopedia of Industrial Chemistry. 2006
- Filtration. Ullmann's Encyclopedia of Industrial Chemistry. 2006
- **P. FINKEL.** *SÖFW-Journal,* 1996, vol. 122, 543
- Kosmetische Färbemittel. Farbstoffkommission der Deutschen Forschungsgemeinschaft. Verlag Chemie, 1984, 81-106